# NOUVEAU FASCICULE DE BREVET EUROPEEN

(11) **EP 0 994 710 B2**
(45) Date de publication et mention de la décision concernant l'opposition: **21.09.2005**
(45) Mention de la délivrance du brevet: 25.09.2002
(21) Numéro de dépôt: 98931849.8
(22) Date de dépôt: 02.07.1998
(51) Int. Cl.: A61K 31/495, A61K 9/00, A61K 47/40

(54) **COMPOSITIONS PHARMACEUTIQUES ADMINISTRABLES PAR VOIE ORALE, COMPRENANT UNE BENZHYDRYLPIPERAZINE ET UNE CYCLODEXTRINE**
ORAL ANZUWENDENDE ARZNEIZUBEREITUNGEN ENTHALTEND EINE BENZHYDRYLPIPERAZINE UND EINE ZYKLODEXTRIN
PHARMACEUTICAL COMPOSITIONS FOR ORAL ADMINISTRATION, COMPRISING A BENZHYDRYLPIPERAZINE AND A CYCLODEXTRIN

(30) Priorité: 03.07.1997 BE 9700572
(43) Date de publication de la demande: 26.04.2000
(73) Titulaire: UCB, S.A., 1070 Bruxelles (BE)
(72) Inventeur: FANARA, Domenico, B-4520 Wanze (BE); BERWAER, Monique, B-4350 Remincourt (BE); NOLF, Philippe, B-1083 Bruxelles (BE); VRANCKX, Henri, B-1190 Bruxelles (BE); DELEERS, Michel, B-1630 Linkebeek (BE)
(74) Mandataire: Lechien, Monique
(86) Numéro de dépôt international: PCT/BE1998/000100
(87) Numéro de publication internationale: WO 1999/001133

(56) Documents cités:
- EP-A- 0 058 146
- EP-A- 0 399 902
- EP-A- 0 605 203
- WO-A-94/06429
- JP-A- 63 243 031
- DATABASE WPI Week 8551 Derwent Publications Ltd., London, GB; AN 85-319295 [51] XP002058687 & JP 60 204712 A (SS PHARMACEUTICAL KK) 16 octobre 1985

## Description

La présente invention concerne des compositions pharmaceutiques administrables par voie orale, comprenant une substance active appartenant à la famille des benzhydrylpipérazines substituées et une cyclodextrine.

De nombreuses substances appartenant à la famille des benzhydrylpipérazines substituées sont connues comme étant des substances ayant des propriétés pharmacologiques intéressantes.

Par exemple, le brevet GB 817231 au nom de la demanderesse décrit des benzhydrylpipérazines substituées répondant à la formule générale 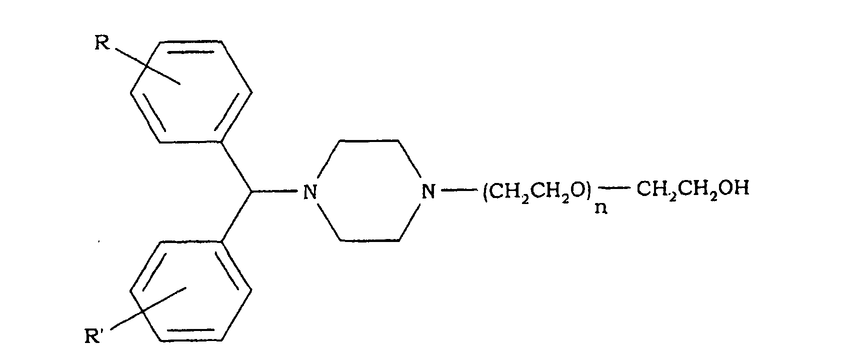 dans laquelle R et R¹ représentent indépendamment l'un de l'autre un atome d'hydrogène ou d'halogène, un groupe alkyle ou alkoxy, R et R¹ pouvant être en position ortho, méta ou para, et n représente le chiffre 1 ou 2, ainsi que leurs sels pharmaceutiquement acceptables.

Parmi ces composés, on retrouve notamment le 2-[2-[4-[(4-chlorophényl)phénylméthyl]-1-pipérazinyl]éthoxy] éthanol, aussi connu sous le nom d'hydroxyzine, et son dichlorhydrate, bien connus pour leurs propriétés antihistaminique et tranquillisante.

Le brevet EP 58146 au nom de la demanderesse décrit des benzhydrylpipérazines substituées répondant à la formule générale 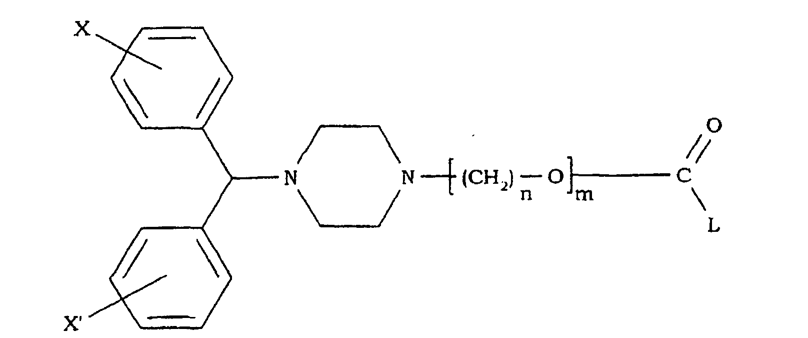 dans laquelle L représente un groupe -OH ou -NH₂, X et X', pris isolément, représentent un atome d'hydrogène, un atome d'halogène, un radical alkoxy linéaire ou ramifié en C₁ ou C₄, ou un radical trifluorométhyle, m est égal à 1 ou 2, n est égal à 1 ou 2, ainsi que leurs sels pharmaceutiquement acceptables.

Parmi ces composés, l'acide 2-[2-[4-[(4-chlorophényl)phénylméthyl]-1-pipérazinyl]éthoxy]-acétique, aussi connu sous le nom de cétirizine, et son dichlorhydrate sont bien connus pour leurs propriétés antihistaminiques.

Jusqu'à présent, les seules compositions pharmaceutiques commerciales administrables par voie orale contenant ce type de composés sont de type conventionnel. Dans le cas de comprimés pelliculés, l'administration se fait par avalement grâce à l'absorption simultanée de liquide. Lorsque l'absorption doit se faire sans absorption simultanée de liquide (conditions pré ou postopératoires, absence d'eau potable, etc.), un mode conventionnel d'administration ne convient pas en raison du goût extrêmement amer de ces benzhydrylpipérazines substituées.

Différentes techniques destinées au masquage de goût de substances pharmaceutiques ont été décrites.

Par exemple, le brevet US 3558600 décrit une méthode pour masquer le goût amer d'antihistaminiques appartenant à la famille des 1-(p-chlorobenzhydryl)-pipérazines substituées, qui consiste à transformer la substance active sous forme de base libre en son sel d'un sulfate d'alkyle à chaîne longue, tel que le sulfate de stéaryle, par exemple.

Une autre méthode connue pour le masquage de goût de principes actifs consiste à former un complexe d'inclusion entre le principe actif et une cyclodextrine. Dans ce cas, le masquage du goût provient de l'emprisonnement du principe actif qui ne peut se libérer lors du passage dans la bouche. Toutefois, cette solution au problème du masquage de goût entraîne un autre problème particulier au masquage du goût de substances pharmaceutiquement actives administrées par voie orale, à savoir, le problème de la biodisponibilité et de la vitesse d'action du principe actif. En effet, si la constante d'association du complexe d'inclusion est trop grande, le principe actif risque de ne pas se libérer suffisamment facilement pour permettre une bonne absorption dans le tractus gastro-intestinal. Dans ce cas, l'effet thérapeutique escompté ne peut pas être obtenu.

Le brevet EP 399902 mentionne ce double problème propre aux compositions pharmaceutiques administrables par voie orale, à savoir le masquage de goût associé à une bonne biodisponibilité. Ce brevet décrit des formes pharmaceutiques lyophilisées et poreuses comprenant, outre les excipients et additifs conventionnels pour ce type de formulation, le principe actif et une cyclodextrine, ainsi que des procédés de préparation de ces formes pharmaceutiques. Des compositions pharmaceutiques contenant les principes actifs suivants sont décrites dans les exemples de réalisation de l'invention: kétoprofène, trimipramine méthanesulfonate, zopiclone, phénobarbital, vitamine A, essence de citron, pritinamycine ou vitamine D3.

Toutefois, ce document ne permet pas de conclure que le masquage de goût et la biodisponibilité de ces principes actifs sont bien obtenus dans tous les cas. Dans le cas des substances pharmaceutiques appartenant à la famille des benzhydrylpipérazines substituées, ce problème revêt une importance particulière, puisque, bien qu'il soit désirable de masquer le goût amer extrêmement désagréable de ces principes actifs, il est aussi indispensable qu'ils soient libérés immédiatement après administration pour obtenir un effet rapide et efficace.

La demanderesse s'est donc fixé comme objectif de rechercher de nouvelles compositions pharmaceutiques permettant une administration par voie orale des substances pharmaceutiques appartenant à la famille des benzhydrylpipérazines substituées plus aisée que ne le permettent les compositions actuelles, tout en assurant une bonne biodisponibilité de la substance active.

Nous venons maintenant de découvrir de nouvelles formes pharmaceutiques administrables par voie orale qui permettent à la fois de masquer efficacement le goût des substances appartenant à la famille des benzhydrylpipérazines substituées et d'obtenir une bonne biodisponibilité de ces composés lors de leur administration par voie orale, même sans prise de liquide simultanée. En particulier, la demanderesse s'est fixé comme objectif de rechercher de telles formulations se présentant sous la forme de comprimés mâchables, de sirops secs, de granulés ou de comprimés sublinguaux.

C'est pourquoi la présente invention concerne des compositions pharmaceutiques solides administrables par voie orale comprenant une substance active appartenant à la famille des benzhydrylpipérazines substituées et au moins une cyclodextrine, la cyclodextrine n'étant pas sous la forme d'un complexe d'inclusion avec la substance active.

Les cyclodextrines utilisables selon la présente invention peuvent être choisies parmi les cyclodextrines α, β ou γ, ou parmi les dérivés alkylés ou hydroxyalkylés de ces dernières, tels que l'heptakis(2,6-di-o-méthyl)-β-cyclodextrine (couramment abrégée DIMEB), la β-cyclodextrine méthylée aléatoirement (couramment abrégée RAMEB) et l'hydroxypropyl β-cyclodextrine (couramment abrégée HPβCD).

Parmi les substances actives appartenant à la famille des benzhydrylpipérazines substituées, nous citerons enparticulierl'acide2-[2-[4-[(4-chlorophényl)phénylméthyl]-1-pipérazinyl]éthoxy]-acétique(cétirine),le2-[2-[4-[(4-chlorophényl)phénylméthyl]-1-pipérazinyl]éthoxy]éthanol (hydroxyzine), l'acide 2-[2-[4-[bis(4-fluorophényl)méthyl]-1-pipérazinyl]éthoxy]acétique (éflétirizine), la 1-[(4-chlorophényl)phénylméthyl]-4-[(3-méthylphényl)méthyl]pipérazine (méclizine) ou la 1-[(4-tert-butylphényl)méthyl]-4-[(4-chlorophényl)phénylméthyl]pipérazine (buclizine), leurs isomères op-tiquement actifs ainsi que leurs sels pharmaceutiquement acceptables.

Les compositions pharmaceutiques selon la présente invention peuvent se présenter sous diverses formes administrables par voie orale. En particulier, les compositions pharmaceutiques selon la présente invention peuvent se présenter sous forme de sirops secs, de comprimés màchables, de granulés ou de comprimés sublinguaux qui se prêtent particulièrement bien à une administration par voie orale sans absorption simultanée de liquide.

Les excipients utilisés sont les excipients conventionnels utilisés pour ce genre de compositions.

Dans le cas de sirops secs et de granulés, on peut utiliser par exemple des diluants comme les polyols (mannitol, sorbitol, saccharose ...) et des arômes.

Dans le cas des comprimés mâchables, on peut utiliser tout excipient classique fournissant de bons paramètres de compression tels que des diluants (mannitol, sorbitol..), des agents de délitement ou gonflants (polyvinylpolypyrrolidone, croscarmellose sodique, amidons et dérivés, cellulose et dérivés...), des agents lubrifiants (stéarate de magnésium...), des agents d'écoulement (aérosil 200...) et des arômes.

Dans le cas de comprimés sublinguaux, on peut utiliser les excipients cités ci-dessus en choisissant parmi ceux qui sont hydrosolubles.

En ce qui concerne les méthodes de préparation, toutes les méthodes courantes utilisées par les pharmaciens pour la préparation de ce type de compositions peuvent utilisées.

La substance active et la cyclodextrine peuvent être simplement mélangées avec les autres excipients et adjuvants.

Les exemples qui suivent illustrent la présente invention sans la limiter. Dans ces exemples, les parties sont exprimées en poids.

### Exemple 1 - Test d'amertume.

Différentes solutions sont préparées en ajoutant la β-cyclodextrine à une solution de 2 mg/ml de dichlorhydrate de cétirizine de telle sorte que le rapport molaire entre la β-cyclodextrine et la cétirizine soit respectivement de 0, 0,5, 1,0, 2,0 et 4,0.

L'amertume de ces solutions a été testée sur un groupe de 7 personnes. Les résultats de ce test sont présentés dans le Tableau 1.

**Tableau 1 -**

| Test d'amertume | | | | | |
|---|---|---|---|---|---|
| Rapport molaire | | | | | |
| β-CD/cétirizine | 0,0 | 0,5 | 1,0 | 2,0 | 4,0 |
| Absence d'amertume 0 | 0 | 0 | 3 | 7 | |
| Amertume très faible 0 | 1 | 6 | 4 | 0 | |
| Amertume marquée | 7 | 6 | 1 | 0 | 0 |

On remarque une réduction de l'amertume du dichlorhydrate de cétirizine lorsque la β-cyclodextrine est ajoutée à la solution de dichlorhydrate de cétirizine. Cette réduction est particulièrement remarquable lorsque le rapport molaire entre la β-cyclodextrine et le dichlorhydrate de cétirizine est compris entre 1,0 et 4,0.

### Exemple 2 - Test de solubilité.

La solubilité de molécules hydrophobes dans l'eau est accrue en présence de cyclodextrines, à la fois en ce qui concerne la vitesse de solubilisation et la quantité de substance active solubilisée. La modification de la solubilité dans l'eau d'une substance active hydrophobe en présence de cyclodextrine constitue donc une méthode couramment utilisée pour mettre en évidence la formation d'un complexe d'inclusion (voir J. SZETLI, dans V.F. SMOLEN and L.A. BALL, Controlled Drug Bioavailability, Vol. 3, Wiley, New York (1985), 365-420).

Bien que le dichlorhydrate de cétirizine soit bien soluble dans l'eau à pH neutre, sa solubilité est beaucoup plus faible lorsque le pH est compris entre 2,5 et 3,5 (solubilité de l'ordre de 1 g/100 ml). Dans ce test, on a examiné la modification de la solubilité du dichlorhydrate de cétirizine dans l'eau à pH 3,4 en présence de β-cyclodextrine, afin de mettre en évidence la formation d'un complexe d'inclusion entre la cétirizine et la β-cyclodextrine.

Deux solutions A et B ont été préparées. La solution A contenait le dichlorhydrate de cétirizine dans de l'eau à pH 3,4; la solution B contenait le dichlorhydrate de cétirizine et la β-cyclodextrine dans un rapport molaire 1:1 dans de l'eau à pH 3,4. Ces deux solutions ont été agitées à température ambiante jusqu'à ce que l'équilibre thermodynamique ait été atteint.

Après agitation, on ne parvient à dissoudre dans la solution A qu'une quantité très faible de cétirizine (1 g/100 ml d'eau). La solution B, quant à elle, a permis de solubiliser 27 g/100 ml de cétirizine dans la phase aqueuse.

D'autre part, la β-cyclodextrine est peu soluble dans l'eau (1,85 g/100 ml). Sa solubilité augmente au fur et à mesure que l'on ajoute du dichlorhydrate de cétirizine, jusqu'à un rapport molaire de β-cyclodextrine/cétirizine de 1: 1. A pH 3,4, la solubilité de la β-cyclodextrine augmente par un facteur d'au moins 30.

### Exemple 3 - Mise en évidence de la formation d'un complexe par spectroscopie UV.

La complexation d'un hôte par une cyclodextrine se traduit en général par un léger déplacement du maximum d'absorption en spectroscopie UV et/ou par une modification du coefficient d'extinction molaire (J. SZETLI dans Cyclodextrin Technology, Chapter 2.2.4.2, Kluwer Academic Publishers, 1988).

Différentes solutions contenant des rapports molaires différents de dichlorhydrate de cétirizine/ β-cyclodextrine ont été préparées, et les différences d'absorbance à 230 nm ont été déterminées. En effet, la cétirizine dans l'eau présente un maximum d'absorption à 230 nm en absence de cyclodextrine.

On constate une diminution de l'absorbance au maximum d'absorption au fur et à mesure que la concentration de β-cyclodextrine augmente. Cet effet hypochromatique indique la formation d'un complexe d'inclusion.

### Exemple 4 - Compétition pour la complexation avec des indicateurs colorés.

Dans cet exemple, on observe les modifications du spectre d'absorption dans le visible d'une solution contenant un complexe entre une cyclodextrine et un indicateur coloré lorsque la cétirizine est introduite dans la solution. Dans ce cas, la cétirizine entre en compétition avec l'indicateur coloré pour la formation d'un complexe d'inclusion. Les modifications du spectre dans le visible permettent donc de déterminer si la cétirizine forme un complexe d'inclusion avec la cyclodextrine.

Deux indicateurs acide-base ont été utilisés: cristal violet et méthyl orange. Dans le cas des indicateurs acide-base, les modifications du spectre d'absorption dues à la complexation par une cyclodextrine sont souvent importantes étant donné que la complexation provoque une modification du pK de l'indicateur. Si le pH de la solution est proche du pK, l'addition d'une cyclodextrine à une solution d'indicateur acide-base provoque l'ionisation ou la désionisation de l'indicateur, ce qui se traduit par une modification de la couleur de la solution. Par conséquent, le maximum d'absorption du spectre visible se déplace en fonction du degré de complexation.

Lorsqu'on introduit le dichlorhydrate de cétirizine dans une solution aqueuse contenant un indicateur acide-base et la β-cyclodextrine, on observe également un déplacement du maximum d'absorption, indiquant par là qu'une partie de l'indicateur n'est plus complexé par la β-cyclodextrine. Ceci signifie qu'une partie de la β-cyclodextrine a servi à complexer la cétirizine introduite dans le milieu. (J. SZETLI dans Cyclodextrin Technology, Chapter 2.2.4.1, Kluwer Academic Publishers, 1988).

On détermine une valeur moyenne de la constante d'association de 3292 mol⁻¹ pour la compétition avec le cristal violet et de 3587 mol⁻¹ pour la compétition avec le méthyl orange.

### Exemple 5 - Identification de la formation d'un complexe par RMN du proton.

La spectroscopie par résonance magnétique nucléaire (RMN) est couramment utilisée pour mettre en évidence la formation de complexes d'inclusion avec les cyclodextrines (F. DJEDAINI et B. PERLY dans D. DUCHENE, New Trends in Cyclodextrin and Derivatives, Chap. 6, § 2&3, Edition de Santé, Paris 1991, F. DJEDAINI et al., J. Pharm. Sciences, 79 (7), 643-646 (1990)).

Dans ce test, différentes solutions contenant des rapports molaires variables de β-cyclodextrine/dichlorhydrate de cétirizine dans un mélange H₂O/D₂O 9:1 ont été analysées par spectroscopie RMN du proton. Les régions du spectre observées correspondent à la zone de fréquence de résonance des protons 2 à 6,6' (d = 3,0 à 4,0 ppm) de la β-cyclodextrine et à la zone de fréquence de résonance des protons aromatiques de la cétirizine (d = 7,2 à 7,6 ppm).

Un seul pic de résonance à une fréquence de résonance moyenne entre la fréquence de résonance de la molécule libre et celle de la molécule complexée est observé pour chaque proton. Cela signifie que le système analysé est en régime d'échange plus rapide que l'échelle de temps de la mesure RMN.

Lorsque la quantité de cétirizine présente en solution avec la β-cyclodextrine augmente, on observe un déplacement important vers les champs élevés pour les protons situés à l'intérieur de la cavité hydrophobe de la β-cyclodextrine (protons 3 et 5). Par contre, les fréquences de résonance des protons situés à l'extérieur de la cavité de la β-cyclodextrine (protons 2 et 4) ne se déplacent pratiquement pas. Ceci démontre clairement la formation d'un complexe d'inclusion dans la cavité de la β-cyclodextrine.

En ce qui concerne les protons de la cétirizine, on constate que seuls les protons aromatiques subissent un déplacement de leur fréquence de résonance. L'interprétation complète est compliquée par le recouvrement des signaux de résonance des 9 protons aromatiques. Cette observation indique l'inclusion de la partie aromatique de la cétirizine dans la cavité de la β-cyclodextrine.

En outre, le coefficient de stoechiométrie du complexe a été déterminée par la technique de la variation continue aussi appelée "méthode de Job" (voir F. DJEDAINI et al., J. Pharm. Sciences, 79 (7), 643-646 (1990), P. JOB, Ann. Chim., 9, 113-134 (1928)). La variation du shift chimique pour le proton 3 de la β-cyclodextrine a été prise comme variable. Par cette méthode, on détermine que le complexe formé a une stoechiométrie 1:1.

### Exemple 6 - Comprimés mâchables de cétirizine à base de polyols (Pour illustration, ne fait pas partie de l'invention)

Le dichlorhydrate de cétirizine (10 parties) et la β-cyclodextrine (55 parties) sont malaxées en présence d'eau dans un mélangeur planétaire pendant 20 minutes. De cette manière, on forme le complexe entre le dichlorhydrate de cétirizine et la β-cyclodextrine. Ce mélange est ensuite séché en étuve.

Après séchage, le complexe est mélangé avec les excipients suivants: Sorbitol (29,45 parties, Acesulfam K (0,7 parties) Aerosil 200 (0,3 parties), Croscarmellose Na (2,1 parties), Glycamil (1,2 partie), arôme de réglisse (0,25 partie).

Le mélange est ensuite comprimé de manière conventionnelle.

### Exemple 7 - Comprimés mâchables de cétirizine sans polyols (Pour illustration, ne fait pas partie de l'invention)

Le complexe dichlorhydrate de cétirizine et de β-cyclodextrine est préparé de la même manière que dans l'exemple 6. Les excipients utilisés sont les suivants:
Polyvinylpolypyrrolidone (35 parties), Avicel pH 101 (50 parties), Avicel CE 15 (7 parties), Aerosil 200 (1 partie), Stéarate de magnésium (1,6 partie), Acesulfam K (1,4 partie), Arômes (2,7 parties).

### Exemple 8 - Sirop sec de cétirizine.

Deux compositions A et B ont été préparées en mélangeant les ingrédients repris dans le Tableau:

| Constituant (en parties) | A | B |
|---|---|---|
| Cétirizine.2HCl | 5 | 10 |
| β-cyclodextrine | 27,5 | 55 |
| Arôme | 0,5 | 0,5 |
| Mannitol | q.s. ad 1000 | q.s. ad 1000 |

Le mélange est granulé avec de l'eau en mélangeur planétaire, puis extrudé. L'extrudat obtenu est séché en lit d'air fluidisé.

### Exemple 9 - Granulés d'hydroxyzine

Une composition C a été préparée en mélangeant les ingrédients repris dans le Tableau:

| Constituant (en parties) | C |
|---|---|
| Hydroxyzine.2 HCl | 25 |
| β-cyclodextrine | 142 |
| Arôme | 2 |
| Saccharose impalpable | q.s. ad 1000 |

Le mélange est granulé avec de l'eau en mélangeur planétaire, puis extrudé. L'extrudat est séché en lit d'air fluidisé.

## Revendications

1. Composition pharmaceutique administrable par voie orale qui se présente sous la forme d'un comprimé mâchable, d'un sirop sec, de granulés ou d'un comprimé sublingual, comprenant une substance active appartenant à la famille des benzhydrylpipérazines substituées, **caractérisée en ce que** la composition pharmaceutique contient au moins une cyclodextrine, la cylodextrine n'étant pas sous la forme d'un complexe d'inclusion avec la substance active.

2. Composition pharmaceutique selon la revendication 1, **caractérisée en ce que** la substance active est choisie parmi le groupe constitué de la cétirizine, l'hydroxyzine, l'éflétirizine, la méclizine et la buclizine, leurs isomères optiquements actifs et leurs sels pharmaceutiquement acceptables.

3. Composition pharmaceutique selon la revendication 1 ou 2, **caractérisée en ce que** la cyclodextrine est choisie parmi le groupe constitué des cyclodextrines α, β ou γ et des dérivés alkylés ou hydroxyalkylés de ces dernières, tels que l'heptakis(2,6-di-o-méthyl)- β-cyclodextrine, la β-cyclodextrine méthylée aléatoirement et l'hydroxypropyl β-cyclodextrine.

4. Composition pharmaceutique selon l'une quelconque des revendications 1 à 3, **caractérisée en ce que** le rapport molaire entre la cyclodextrine et la substance active est compris entre 1,0 et 4,0.

## Patentansprüche

1. Oral verabreichbare pharmazeutische Zusammensetzung, die in Form einer kaubaren Tablette, eines trockenen Sirups, von Granulat oder einer Sublingualtablette vorliegt, und die einen Wirkstoff umfasst, der der Familie der substituierten Benzhydrylpiperazine angehört, **dadurch gekennzeichnet, dass** die pharmazeutische Zusammensetzung wenigstens ein Cyclodextrin enthält, wobei das Cyclodextrin nicht in Form eines Einschlusskomplexes mit dem Wirkstoff vorliegt.

2. Pharmazeutische Zusammensetzung gemäß Anspruch 1, **dadurch gekennzeichnet, dass** der Wirkstoff ausgewählt ist aus der Gruppe, die besteht aus Cetirizin, Hydroxyzin, Efletirizin, Meclizin und Buclizin, ihren optisch aktiven Isomeren und ihren pharmazeutisch annehmbaren Salzen.

3. Pharmazeutische Zusammensetzung gemäß Anspruch 1 oder 2, **dadurch gekennzeichnet, dass** das Cyclodextrin ausgewählt ist aus der Gruppe, die besteht aus α-, β- oder γ-Cyclodextrinen und alkylierten oder hydroxyalkylierten Derivaten dieser Letzteren, wie Heptakis (2,6-di-o-methyl)-β-cyclodextrin, statistisch methyliertem β-Cyclodextrin und Hydroxypropyl-β-cyclodextrin.

4. Pharmazeutische Zusammensetzung gemäß einem der Ansprüche 1 bis 3, **dadurch gekennzeichnet, dass** das Molverhältnis zwischen dem Cyclodextrin und dem Wirkstoff zwischen 1,0 und 4,0 liegt.

## Claims

1. Pharmaceutical composition which can be administered orally in the form of a chewable tablet, a dry syrup, granules or a sublingual tablet, comprising an active substance belonging to the family of substituted benzhydryl piperazines, **characterised in that** the pharmaceutical composition contains at least one cyclodextrin, the cyclodextrin not being in the form of an inclusion compound with the active substance.

2. Pharmaceutical composition as claimed in claim 1, **characterised in that** the active substance is selected from the group consisting of cetirizine, hydroxyzine, efletirizine, meclizine and buclizine, their optically active isomers and their pharmaceutically acceptable salts.

3. Pharmaceutical composition as claimed in claim 1 or 2, **characterised in that** the cyclodextrin is selected from the group consisting of •, • or • cyclodextrins and alkylated or hydroxyalkylated derivatives of these, such as heptakis(2,6-di-o-methyl)-•-cyclodextrin, randomly methylated •-cyclodextrin and hydroxypropyl •-cyclodextrin.

4. Pharmaceutical composition as claimed in any one of claims 1 to 3, **characterised in that** the molar ratio between the cyclodextrin and the active substance is between 1.0 and 4.0.
